(19) **Europäisches Patentamt**
**European Patent Office**
**Office européen des brevets**

(11) **EP 2 734 575 B1**

(12) **EUROPEAN PATENT SPECIFICATION**

(45) Date of publication and mention
of the grant of the patent:
**04.09.2019 Bulletin 2019/36**

(51) Int Cl.:
***C08J 7/04*** (2006.01)

(21) Application number: **11764857.6**

(22) Date of filing: **22.07.2011**

(86) International application number:
**PCT/IB2011/053271**

(87) International publication number:
**WO 2013/014493 (31.01.2013 Gazette 2013/05)**

(54) **WHEY PROTEIN COATED FILMS**

MIT MOLKEPROTEIN BESCHICHTETE FILME

FILMS REVÊTUS DE PROTÉINE DE PETIT-LAIT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**28.05.2014 Bulletin 2014/22**

(73) Proprietors:
• **Pimec**
**08015 Barcelona (ES)**
• **Magyar Müanyagipari Szövetség (MMSZ)**
**1152 Budapest (HU)**
• **Giz Grozd Plasttehnika**
**3000 Celje (SI)**
• **Lleters De Catalunya, Llet Nostra, SCCL**
**17003 Girona (ES)**
• **Lajovic Tuba Embalaza d.d.**
**1000 Ljubljana (SI)**
• **Meierei-Genossenschaft Langenhorn E.G.**
**25842 Langenhorn (DE)**
• **Dunreidy Engineering Ltd.**
**Kilkenny (IE)**
• **Manufacturas Serviplast, S.A.**
**08907 L'Hospitalet de Llobregat (ES)**

(72) Inventors:
• **SCHMID, Markus**
**85354 Freising (DE)**
• **NOLLER, Klaus**
**85354 Freising (DE)**
• **WILD, Florian**
**85354 Freising (DE)**
• **BUGNICOURT, Elodie**
**E-08860 Castelldefels (Barcelona) (ES)**

(74) Representative: **Alier Benages, Elisabet**
**Consell de Cent, 417-419, 2-1.**
**08009 Barcelona (ES)**

(56) References cited:
**US-A- 5 543 164**

• **KIRSTEN L. DANGARAN ET AL: "Kinetics of
Sucrose Crystallization in Whey Protein Films",
JOURNAL OF AGRICULTURAL AND FOOD
CHEMISTRY, vol. 54, no. 19, 1 September 2006
(2006-09-01), pages 7152-7158, XP55023736,
ISSN: 0021-8561, DOI: 10.1021/jf0611670**
• **M.B PEREZ-GAGO, P. NADAUD, J.M KROCHTA:
"Water vapor permeability, solubility, and tensile
properties of heat-denatured versus native whey
protein films", JOURNAL OF FOOD SCIENCE, vol.
64, no. 6, 1999, pages 1034-1037, XP002673368,**
• **H. JOOYANDEH: "Whey protein films and
coatings, a review", PAKISTAN JOURNAL OF
NUTRITION, vol. 10, no. 3, June 2011 (2011-06),
pages 296-301, XP002673367, ISSN: 1680-5194**

## Description

The present invention relates to the plastic field, particularly to the use of whey-protein films in multilayer films for packaging applications.

BACKGROUND ART

Paper, board and currently available polymers and/or biopolymer films are mostly used in combination with barrier materials derived from oil based plastics or aluminum to enhance their low barrier properties. In order to replace these non renewable materials, current research efforts are focused on the development of sustainable coating while still maintaining the functional properties of the resulting packaging materials.

Especially in the food industry high demands are put on packaging material in order to preserve the quality of the packed good throughout its lifecycle. The requirements for a packaging material are specific to the type of good to be packed. Materials need to fulfill different barrier to light, moisture, water vapour and gases. Appropriate levels of oxygen, and carbon dioxide, required packing atmosphere, respiration rate and thus optimally preserve the packed food, avoid colour or taste deviation, oxidation of grease, formation of microorganisms, damaging of nutrients, etc. have to be taken into account.

To achieve these requirements expensive multilayer co-extruded or laminated plastic films are widely used in the packaging industry whereby ethylene vinyl alcohol copolymers (EVOH) are often used to reach sufficient oxygen barrier. Polymers used for those applications are petroleum based and there combination in various layers hampers recyclability as mono-materials of high purity are needed for reprocessing. Thus research into sustainable packaging materials still maintaining the performances of composite structures has been recently intensified.

Whey is a by-product of cheese manufacturing that contains approximately 7% dry matter. In general the dry matter includes 13% proteins, 75% lactose, 8% minerals, about 3% organic acids and less than 1% fat. Two main kind of whey exist:

Sweet whey, with a pH of approximately 6.0, originates from rennet-coagulated cheese production such as Cheddar.
Sour whey, with a pH approximately 4.6, results from the manufacture of acid-coagulated cheese.

The technical definition for whey protein, generally known by the skilled person in the art, is "those that remain in the milk serum after coagulation of the caseins at pH 4.6 and temperature 20 °C".

Whey proteins are a mixture of proteins with numerous and diverse functional properties and therefore have many potential uses in food applications. The major whey proteins are $\beta$-Lactoglobulin ($\beta$-Lg) and $\alpha$-Lactalbumin (a-La). They represent approximately 70 % of the total whey proteins and are responsible for the hydration, gelling and surface-active properties of the whey protein ingredients. The other major proteins are Bovine Serum Albumin (BSA) and Immunoglobulin (Ig).

Whey proteins are soluble over a wide range of pH. However, various combinations of pH, temperature, and mineral composition induce selective denaturation, aggregation, and precipitation of whey proteins. In general, whey proteins are heat-labile proteins. Heat decreases their stability in the following order: $\alpha$-La> $\beta$-Lg>BSA>Ig. Thermal denaturation and heat gelation of whey proteins are important functional characteristics in numerous products. The stability of the tertiary structure of whey proteins is determined by various non-covalent interactions and by the disulfide bonds, which are formed by two cysteine residues. $\beta$-Lg has two internal disulfide bonds and one free thiol group, while $\alpha$-La has eight cysteine groups that are all involved in internal disulfide bonds. The first step during heat treatment involves a preliminary change in the conformational structure of protein, the denaturation step. The second and distinctly different step involves the process of aggregation, and this may be followed by coagulation or gelation. Denaturation has been defined as a major change of the very specific native protein structure, without alteration of the amino acid sequence. Thus changes are restricted to those occurring in secondary or higher-order structure.

In protein-based films, protein-protein interactions determine the characteristics of the film. Film-forming ability may be influenced by amino acid composition, distribution, and polarity, conditions necessary for ionic crosslinks between amino and carboxyl groups, hydrogen bonding groups, and intramolecular and intermolecular disulfide bonds.

Native and heat-denatured films differ in physical structures. Native whey proteins are globular proteins with most hydrophobic and sulfhydryl groups turned to the interior of the molecule. Heat denaturation of the whey proteins induces protein unfolding and exposure of internal sulfhydryl groups, promoting intermolecular disulfide bond formation. Such differences influence the molecular structure of the final film. Thus, heat denatured whey-protein films are made of cross-linked protein strands, whereas native whey-protein films have a more random structure in which cohesion is mainly due to hydrogen bonding. These different structures results in different permeability properties of the resulting films.

Since $\beta$-Lg is the dominant whey protein, it tends to control the thermal behaviour of the total whey protein system. This protein has already been subject to many studies and it is generally accepted that thiol/disulphide exchange reactions, leading to the formation of intermolecular disulphide bonds, play a significant role in the heat-induced denaturation and aggregation of β-Lg. Heat-induced denaturation and aggregation of whey proteins may result in the formation of a gel, depending on experimental conditions such as protein concentration, pH, presence and concentration of salts and heating temperature

Various authors have reported work done at academic level dealing with the properties of the coated plastic film. Thus, authors have reported the good barrier properties of whey proteins when they have undergone to a pre-denaturation step, especially for their use as coatings on paper but also on plastic substrates. Indeed whey coatings on polypropylene (PP), polyvinylchloride (PVC) and low density polyethylene (LDPE) performed excellent visual properties, like excellent gloss and high transparency, as well as good mechanical properties. Nevertheless, all the other requirements put on food packaging such as food contact, post processability were never taken into account, and in general it stopped with a bilayer (coated film) and not a full laminate.

The determination of the barrier properties of a polymer is crucial to estimate and predict the shelf-life of the packed food. Oxygen-barrier layers in food packaging materials typically consist of expensive synthetic barrier polymers including ethylene vinyl alcohol (EVOH) copolymers, polyvinylidene chloride (PVDC), polyethylene terepthalate (PET), and polyamide-6 (nylon), which are commonly used in the form of coextruded or laminated films and coatings. Water vapour and oxygen are two of the main permeants studied in packaging applications, because they may transfer from the internal or external environment through the polymer package wall, resulting in a continuous change in product quality and shelf-life. The oxygen barrier is quantified by the oxygen permeability coefficients (OPC) which indicate the amount of oxygen that permeates per unit of area and time in a packaging material.

In J Agricultural and Food Chem 2006, 54/19: 7152-7158 it is analyzed the kinetic of crystallization of sucrose in whey protein isolate films in different relative humidity environments in presence of different crystallization inhibitors.

In J Food Sci 1999, 64/6: 1034-1037 it is disclosed the water vapor permeability, solubility in water and mechanical properties of heat-denatured whey protein films and native whey protein films.

In Pakistan J of Nutr 2011, 10/3: 296-301 it is mentioned that different drying conditions can change edible film morphology which affects appearance, barrier and mechanical properties. And it is highlighted that although solvent cast whey protein films can form from native proteins, their film network can be improved and the resulting solvent-cast film tensile and barrier properties improved through heat denaturation and cross-linking of the whey protein chains.

US5543184 discloses a method for preparing a protein based water insoluble coating for a food item. The method comprising treating an aqueous solution of a protein to obtain a denatured protein solution and then applying said denatured protein solution to a food item and drying to form a coating for said food item.

In J Dairy Sci 2014, 97: 5315-5327 it is demonstrated that, regarding coating derived from 0:100 (D:N) WPI ratios and dried at different temperature-time profiles, as TSM was almost 100 % in all cases. In this document it is confirmed that when a substrate coated with a native whey-protein is dried at a temperature ranged between 35-140°C no complete denaturation of the protein occurs.

In Critical Rev in Food Sci and Nutrition 1993. 33:6 431-476 it is stated that both, WPI and WPC differ mainly in composition: WPI contains a higher protein and proportionately lower concentrations of lactose and minerals than WPC.

Therefore, there is a need for a biodegradable and easily recyclable film while keeping high barrier properties which can be obtained and applied by standard processes in the plastics sector, and more specifically in the packaging sector. The films should be usable in multilayer constructions with standard thermoplastic materials during the production process, and also enable a good adhesion on these common substrates. Additionally, the films must show good barrier properties, as well as adhesion, mechanical properties, permeability, good processability (deformability and deformation speed rate).

SUMMARY OF THE INVENTION

The present invention relates to the development of barrier layers or film based on whey protein to be used in multilayer-films principally for food packaging. Although, taking into account the properties obtained for the material, its use is considered also in other application such as pharmaceutical and cosmetics packaging.

The inventors have found that it is possible to carry out an efficient industrial process for the manufacture of a whey-protein coated substrate film for packaging by using native whey protein during the coating step, whereas denaturation of the protein occurs during the drying step.

Native whey protein formulations can be applied with higher solid contents compared to fully denatured whey protein formulations, leading to solutions which can easily be used for the coating process in all classic process types (i.e., gravure, spraying, comma, curtain and multi-roller applications). Furthermore, the simultaneous denaturation and drying (curing and crosslinking) of the coating leads to avoidance of the upstream denaturation process and thereby to a considerable saving of process energy. The result is an additional increase in efficiency because less water has to be evaporated during the drying process.

Therefore, the present invention provides an improved industrial process for the preparation of whey protein-based coated substrate films for packaging, which is cost-effective, consumes less water and saves energy and can be performed using standard coating processes.

Accordingly, a first aspect of the invention relates to a process of preparing a whey-protein coated substrate film for packaging, wherein such manufacturing process comprises the following steps:

a) providing a coating composition containing a water solution of whey protein that is characterised by its partial or complete native state. The whey protein component has at least 40% of its proteins in its native state, the whey protein is selected from the group consisting of a whey protein isolate (WPI), a whey protein concentrate (WPC), and a mixture thereof; and

b) applying directly the coating composition of step a) onto a substrate film in order to obtain a coated substrate film wherein the coating has at least 40% of the whey protein in its native state; and

c) drying it at a temperature between 60-160 °C.

A second aspect of the invention relates to a whey-protein coated substrate obtainable by the process according to the invention.

It is also another aspect of the present invention a packaging film comprising the whey protein coated substrate as defined herein.

Another aspect of the invention relates to the use of the whey protein coated substrate as defined herein as packaging film.

Additionally, another aspect of the invention relates to a food, pharmaceutical or cosmetic product packaged in a packaging film as defined herein.

DETAILED DESCRIPTION OF THE INVENTION:

Substrate

The whey-protein layer is designed to be either an intermediate layer within a composite (multilayer structure) or a surface layer, but in any case it needs to be combined with the structural layer (substrate). In any case, both the intermediate layer within a composite (multilayer structure) and the surface layer are barrier layers. To ensure the retention of modified, vacuum or normal atmosphere during the lifetime of the packaging and protect the packaged product (food stuff, pharmaceutical ingredient, etc) from the exterior, many different substrates are often combined into a structure with several layers, each layer having its own function. Combination of layers is not only to prevent permeation from inside to outside; it's for the other way around also. Different substrates can therefore be chosen and combined to achieve the proper mechanical strength, water vapour barriers, gas barriers, gas penetrability, anti-mist properties and sealing properties. The skilled person in the art would recognize the most suitable substrates to be used in order to achieve the desired properties.

Therefore, according to an embodiment of the invention, the substrate on which the native whey protein is deposited during the coating step can be of different material.

Preferably, the substrate on which the whey-protein could be coated is selected from paper, cardboard, metallic foil, or plastic. More preferably, the substrate is a plastic film, particularly a polymeric film selected from polyethylene terephtalate (PET) or polyolefins such as polypropylene (PP), polyethylene (PE). Although others such as polyester, polystyrene (PS), polyvinyl chloride, nylon, ethylene vinyl acetate and ethylene vinyl alcohol polymer, ethylene vinyl alcohol (EVOH), LDPE/LLDPE, polyvinylidene chloride (PVdC), polylactic acid (PLA), low density polyethylene (LDPE), ethylene vinyl acetate (EVA), crystallized polyethylene terephthalate (CPET), amorphous polyethylene terephthalate (APET), polyvinyl chloride (PVC), poly(butylene adipate-co-terephthalate), high density polyethylene (HDPE) and polyhydroxyalkonoates (PHA) such as polyhydroxybutyrate (PHB) could also be used as substrate materials.

These polymeric films are usually laminated or coextruded with a polymer suitable for heat-sealing (see Table 1) which comes in direct contact with the product.

Following Table 1 reports the primary functions of the most common polymeric materials used in packaging formulations.

**Table 1 - Primary functions of the main materials used as substrate**

| Material | Function |
| --- | --- |
| HDPE, PVDC, PP | Moisture barrier |
| PVDC, EVOH acrylonitrile | $O_2$ barrier |
| HDPE, PP | Elasticity, microwave possibilities |

(continued)

| Material | Function |
|---|---|
| Nylon | High temperature resistance, flexibility, hardness, moulding strength |
| CPET | Elasticity, high temperature, $O_2$ barrier |
| APET | Elasticity, $O_2$ barrier |
| Polyester | High temperature resistance, flexibility puncturing resistance |
| PVC, PET, LDPE, HDPE, EVA | Sealing layers |
| EVA | High $O_2$ and $CO_2$ penetrability |

Whey protein properties

On the other hand, whey is a natural product, though it can come in various forms and from different origins. As a living feedstock, it also has some inherent variability, and storage conditions can also impact its properties.

Protein products can vary in their properties over a wide range depending e.g. on raw material, processing, nativity or pureness. Thus, in the procedure of the present invention it is possible to use not only commercially available WPC and WPI products but also WPC and WPI obtained from sour whey or sweet whey.

The skilled person in the art knows different techniques in order to obtain concentrate and isolate whey proteins from whey. Thus, to concentrate the native proteins from whey a multi-stage membrane filtration and drying process can be used. The microfiltration (e.g. 200 kDa membrane pore size), and the combined ultrafiltration and dia-filtration (e.g. 10 kDa membrane pore size) allow production of whey protein concentrates and isolates. Besides, whey protein isolates can also be purified using ion exchange chromatography.

Generally, the whey protein product suitable for the process of the present invention shows a high degree of pureness, a high dry mass content, high protein content, and high protein nativity degree.

According to an embodiment of the invention, the degree of pureness of the whey protein (measured as the % protein content by dry matter (d.m.)) is preferably between 60-100 % d.m., more preferably 80-99% d.m. most preferably 85-99% d.m. The dry mass content for dried whey powders is usually in the range of 85 to 98 %.

When commercially-available protein WPC or WPI are used in the process of the present invention, they should be obtained in a manner that minimizes denaturing of the whey protein fraction. That is in particular, the whey protein fraction is subjected to minimal high temperature treatment (below 58°C for aqueous solutions, thus avoiding denaturation of the protein) during concentration of the whey which may include the steps of ultrafiltration, evaporation and spray drying.

Nativity

In the context of the present invention, the term "denaturation" refers to thermal induced denaturation of the protein, wherein the native protein have undergone changes in its secondary or higher-order structure as a consequence of a heat treatment.

According to an embodiment of the present invention, the protein nativity degree of the coating solution should be maintained in the range between 40-100%, preferably 65-100%, most preferably 75-100%.during the coating step.

The inventors have observed that, when denatured whey protein is used, whey protein concentration is limited to 10% in denatured application whatever coating process is used. Owing to aggregation of the protein molecules during denaturation, due to disulphide and hydrogen bonds, the volume fraction increases which leads to higher viscosity. WPI concentrations above 10 % result in formation of a gel.

On the contrary, in the process according the present invention, due to the fact that native proteins show lower viscosity it is possible to increase the dry matter content of whey protein solutions. Higher whey protein concentrations are also possible. Furthermore denaturation takes place directly in the dryer of the coating machine which has the advantage that one step in the process, denaturation before coating, is omitted. This is a big advantage regarding ecological (lower energy consumption) as well as economical aspects.

The protein nativity was examined by differential scanning calorimetry using Q 2000 DSC (TA Instruments, New Castle Delaware, USA). Differential Scanning Calorimetry is a thermo analytical technique which measures the necessary energy to increase the temperature of a sample and a reference material as function of temperature. In this analysis the temperature increases linearly during the measurement (heating rate e.g. 10K/min). DSC analysis measures the amount of energy necessary for the unfolding of the protein, due to their denaturation (denaturation enthalpy). By that, endotheric and exothermic reactions (e.g. crystallisation, protein denaturation, starch gelling) in the tested samples can be analysed. For measuring protein nativity of whey protein products, 10 % aqueous protein solutions from the WPC or WPIs at pH 7 are prepared and subsequently analyzed. Therefore samples of the protein solutions (10 mg) were sealed into pans and heated from 23 to 120 °C in the DSC cell. The degree of denaturation is calculated taking as zero denaturation the highest measured denaturation enthalpy, that was measured for a commercially available whey protein isolate: BiPro from Davisco Foods Int., USA with a denaturation enthalpy of 8.2 J/g d.m. In 10% aqueous solutions denaturation peaks appear within the temperature range 65 to 80°C.

A similar method for measuring protein nativity of whey protein in the dried coating can be carried out in order to determine the nativity degree of the final product after drying step. However, denaturation temperatures are higher due to the absence of water. In this case the degree of denaturation is calculated taking as zero denaturation the value of denaturation enthalpy of the dried sheet at 23°C (internal standard).

Furthermore, dissociated whey proteins act as plasticizer. Their ability to bind more water intensifies the plasticizing effect and allows reduction or even complete absence of a plasticizer in the whey proteins coating solution.

Plasticizer

According to an embodiment of the invention, the whey protein solution is mixed with plasticizers in order to improve the thermo-mechanical behaviour of the film. Good processability is mandatory whenever considering a new material. Therefore, deformability and deformation speed rate need to match that of conventional materials at adequate temperatures. The plasticizers, making the film more flexible, will allow tuning the processability of the resulting material.

In the context of the present invention, plasticizers are defined as "substantially non-volatile, high boiling, non-separating substances, which when added to another material change the physical and/or mechanical properties of that material". Plasticizers reduce intermolecular forces like hydrogen bonding and allow better movement of the polymer chains.

The presence of a plasticizer results in a reduction of brittleness and prevention of cracking. Several plasticizers already known in the art could be used. In a preferred embodiment, the plasticizer is selected from polyethylene glycol (PEG), propyleneglycol (PG), glycerol and sorbitol. Being particularly preferred the use of sorbitol or glycerol.

The amount of plasticizer in the composition ranges from 20 - 200 wt. % based on the dry matter content of whey protein. Preferably, between 50-120 wt. %, and more preferably between 66-100 wt.%.

Best results using sorbitol are obtained with an addition to the solution of 80-120 wt. %, based on the dry matter content of whey protein. More preferably, with an addition of 100 wt. % sorbitol. Whereas, using glycerol, best results are obtained adding between 50-80 wt. %, based on the dry matter content of whey protein, to the solution. Preferably, adding 67 wt. % glycerol.

Dry matter

In order to optimize the maximum protein content, it is necessary to determine the optimal dry matter content for each plasticizer type.

In the context of the present invention, the terms "dry matter content" or "solid content" refers to the sum of the protein concentration and the amount of plasticizer together with the content of other minor components such as lactose, mineral salts, etc.

High dry matter content leads to better energy and cost efficiency of the process.

According to an embodiment of the present invention, the coating composition has a dry matter content between 5-75 wt. %. Preferably the dry matter content is between 10-60 wt. %, more preferably 15-50 wt. %, being particularly preferred between 20-45 wt. %.

According to an embodiment of the present invention, the protein content in the coating solution to be applied onto the substrate is up to 40 % w/w, preferably up to 30% w/w, and more preferably up to 20% w/w.

The amount of protein content in the coating solution depends on the plasticiser and the amount thereof. When using sorbitol as plasticiser, the preferred amount of protein concentration is between 15-25 % w/w, being particularly preferred a protein concentration of 20 % w/w in the coating solution, which results in obtaining coated films with similar mechanical properties than with lower concentration.

When using glycerol as plasticiser, the preferred amount of protein concentration is between 20-35 % w/w, being particularly preferred a protein concentration of 25 % w/w in the coating solution.

In summary, the maximum whey protein concentration which could be properly used can be increased when native protein is used instead of denatured protein:

| | |
|---|---|
| Native protein application: | 20 % (Sorbitol as plasticizer) |
| | 25 % (Glycerol as plasticizer) |
| Denatured protein application: | 10 % (the 2 plasticizers) |

Regarding the maximum dry matter content, it also could be increased:

| | |
|---|---|
| Native protein application: | 30 % (Sorbitol as plasticizer) |
| | 34 % (Glycerol as plasticizer) |
| Denatured protein application: | 20 % (Sorbitol as plasticizer |
| | 17 % (Glycerol as plasticizer) |

The total dry matter content in native whey protein formulations can be significantly increased compared to denatured formulations. This is an important fact for industrial processing. Higher dry matter content results in shorter drying time. In addition curing of the proteins directly in the dryer saves an auxiliary processing step. Viscosities of native and denatured formulations strongly differ from each other. This influences the coating system that can be used for industrial application.

Additives

Other, optional, additives can be mixed with the whey protein and the plasticizer. Therefore, according to one embodiment of the present invention, the coating composition of step a) further comprises other additives selected from anti-oxidants, antimicrobials, colorants, pigments, ultraviolet absorbers, antistatic agents, crosslinkers, fillers, oxygens scavengers, humidity absorbers, biocides. and mixtures thereof.

The main focus for the invention is the replacement of synthetic barrier layers, such as EVOH, by whey-protein in multilayer packaging applications while maintaining the high oxygen barrier, as well as other thermo-mechanical properties.

As films get resistant to scratching after a short aging period, industrial applications are feasible even though the whey layer is not protected in a sandwich structure by e.g. a sealing layer. One possibility which might lead to better scratch resistance is incorporation of heavy metal ions. Sulfhydryl groups can be oxidized by these ions as they show high affinity to each other.

This oxidation prevents thiol groups from exchange with disulphide bonds and position rearrangement of those thus leads to additional disulphide bridges.

Whey protein modification

Modifications can be used to adapt the original protein properties to a desired functionality, for example to make film formation more homogenous, and therefore also to prevent the agglomeration and obtain a suitable film building behaviour. These modifications are applied with the proteins' native state maintained.

Thus, according to an embodiment of the present invention, the process further comprises to carry out a protein modification previously to its use in the coating step. The protein modification may be carried out by:

- enzymatic hydrolysis by treatment with a protease enzyme;
- chemical modification by introducing functional chemical groups into the protein molecules, e.g. by acetylation or succinylation;
- physical modification, e.g. with dynamic high pressure.

Enzymatic hydrolysis is used if a reduction of molecular weight is of advantage and highest solubility is required. During enzymatic hydrolysis a severe heating step is required to inactivate the enzymes, therefore, in the case that an enzymatic hydrolysis is carried out, the heating step in order to inactivate the enzymes must be carried out after the coating step, i.e. during drying of the whey-protein coated substrate. Another possibility is, only to incorporate a small amount of enzymatic modified whey protein, so that the total share of native protein maintains above 40% of the total protein amount. According to an embodiment, the realized enzymatic hydrolysis is carried out with the enzyme Alcalase® 2.4 (Novozymes A/S, Bagsvaerd, Denmark), although other protease enzymes currently known in the art could be used.

Physical modification with dynamic high pressure is a milder process leading to dissociation of protein aggregates and partial unfolding of the molecules. Physical modification (e.g. high pressure homogenisation with pressure lower than 2000 bar) has only low impact on protein nativity. High pressure homogenization can improve film building of partially denatured proteins to some extend, however it reduced somewhat agglomeration and may therefore contribute to improved properties or processability.

Chemical modification leads to higher protein denaturation with increasing degree of modification.

Acetylation with acetic anhydride inserts covalent bound neutral acetyl groups to the protein amino group. This results in a partial unfolding of the protein backbone because of reduced electrostatic attraction between oppositely charged amino acid side chains. Practical effects of acetylation may involve a slight increase of aqueous solubility, reduced isoelectric point, and decreased tendency to gel upon heating.

Reaction with succinic anhydride introduces anionic succinate groups covalently linked to the amino groups of lysine. Succinylation generally has greater effects upon protein conformation and functional behavior than acetylation. The electrostatic repulsive forces, resulting from the enhanced negative charge, lead to more extensive unfolding of the polypeptide chain. Alterations of functionality commonly associated with succinylation include increased aqueous solubility, enhanced hydration, and modified surfactant properties. Because of that, enhanced building of homogenous films may be possible.

Since increasing degree of chemical modification in general leads to higher viscosity in aqueous solution, it does not contribute to higher potential dry matter in the initial coating solution. Chemical acylation, both acetylation and succinylation results in a reduction in the agglomeration of proteins and increase transparency of the protein gels. In particular succinylation increases gel strength, thus scratch resistance that may increase mechanical resistance if the whey-protein coating film is used as top layer.

The degree of modification is highly dependent on the added amount of anhydride. With an amount of 5 %, related to the protein mass, a degree of modification of about 55 % was reached for both acetylation and succinylation. A modification degree of about 94 % was achieved by applying 10 % anhydride. The addition of 20 % anhydride led to a degree of modification of approximately 97 %.

Substrate pretreatment

Substrates with polar nature, like PLA, EVOH, can be coated directly. Nevertheless surface of substrates with non-polar nature are not likely to offer binding sites for WPI coatings. Therefore, according to a preferred embodiment, in case of substrates with non-polar nature like PE, the whey-based formulation is coated on the surface of the substrate right after the substrate goes through a surface pre-treatment. The skilled person in the art would recognize the most suitable methods of pre-treatment of the substrate to be used in order to achieve the desired properties. Among others, it is possible to cite the following: corona discharge and plasma treatment. According to a preferred embodiment, the surface pre-treatment is a corona discharge treatment.

Corona discharge, as well as other known surface activation treatments results in an improved wettability, compatibility and adhesion of the substrate.

Corona discharge treatment is a form of plasma treatment: it operates at atmospheric pressure and it is necessary to decrease the surface energy of many plastics including polyolefin films. Decrease the surface energy means increases the wettability and the surface adhesion of the coating.

This process is formed by different parts:

- the film pass over a metal roller which is covered with an insulating material;
- an aluminium metal electrode, usually 2mm far away from the film;
- high frequency generator (10-20 kHz) and step-up transformer which transfer a high voltage (typically 20 kV) to the electrode.

The applied voltage ionizes the air and it becomes plasma. It consists in ions, electrons, excited neutrals and photons in the UV visible region. The current flow comes from the electrode directly to the polymer surface and the oxidation takes place developing the successive introduction of polar functional groups

The effect of corona treatment on the adhesion is the increase of the attractive forces between liquid molecules and the molecules of the substrate surface

During the corona treatment two reactions take place: the production of carbonyl and the production of ether. The first reaction takes place at a faster rate than the second reaction and it is the desired one. The increase of the energy surface is due to the formation, at the beginning of the reactions, of high polar groups such as carbonyl, carboxyl and hydroxyl.

The second reaction is the conversion of these carbonyl groups into ether groups which are non polar groups and this tends to lower the surface energy.

According to a preferred embodiment, the substrate goes through a corona pretreatment in order to achieve sufficient adhesion of the whey coating to the substrate better than 1.5 N/15mm, according to EN ISO 4624:2002.

Solution preparation

During preparation of the coating solution by mixing the water solution of whey-proteins with the plasticizer and, optionally other additives, air bubbles can be formed in the solution. Therefore, according to an embodiment of the present invention, before using the solution for the coating process, the air bubbles should be removed to have a final homogeneous layer deposited of coating on the substrate, e.g. by putting the solution into an ultrasonic bath to break and remove all the air bubbles, or by mixing the components under vacuum.

Manufacturing process specifications

According to an embodiment of the invention, the process comprises additional steps other than mixing the whey-protein with the plasticizer and coating the mixture onto the substrate.

Therefore, according to an embodiment of the present invention, processing properties of the whey protein products were in general improved with increasing protein pureness and maintained protein nativity. Decreasing mineral contents, in particular bivalent ions such as $Ca^{2+}$, results in a reduced aggregation during heating of aqueous protein solution that enables the formation of smooth and fine stranded films. Further, pH values far from the isoelectric point improved film building properties. Influence of NaCl was independent of the nature of the whey protein. Either no salt or very small amounts of NaCl (up to 1%, more preferably up to 0.5%) had a desirable effect.

Therefore, the process according to the present invention may comprise the following steps:

a) optionally

- if raw liquid sour or sweet whey is used as raw material, processing the whey into WPC or WPI with the suitable pureness degree, dry matter content and nativity degree; or
- if commercially available WPC or WPI products are used as raw material, processing it until suitable pureness degree, dry matter content and nativity degree are achieved;

b) optionally

- to carry out a protein modification by a method selected from:

  ∘ enzymatic hydrolysis, wherein the inactivation of the enzyme is carried out during drying step;
  ∘ chemical modification by introducing functional chemical groups into the protein molecules, e.g. by acetylation or succinylation; or
  ∘ physical modification, e.g. with dynamic high pressure.

c)

- preparing a water solution of the WPC or WPI previously obtained and mixing it with a plasticiser;

d) optionally

- adding additional additives selected from anti-oxidants, antimicrobials, colorants, pigments, ultraviolet absorbers, antistatic agents, crosslinkers, fillers, oxygens scavengers, humidity absorbers, biocides, or mixtures thereof;

e) optionally

- removing the air bubbles present in the solution

f) optionally

- undergoing the substrate to a pretreatment, preferably corona treatment, in order to have a good adhesion and compatibility between the whey-protein layer and the substrate, and to increase the wet ability of the substrate;

g)

- coating the composition resulting onto the substrate film, maintaining at least 40% of the whey protein isolate or concentrate in their native state

h) optionally

- drying the whey-protein coated substrate obtained in step g).

The skilled person will be aware of the advantages of carrying out one or more of the optional steps above mentioned, and about the order to perform thereof.

A packaging material including at least one or more layers of the whey-protein coated substrate film that is obtained by the method of the present invention, the packaging material being laminated with another material, is also one of preferred embodiments of the present invention. Although the configuration of this packaging material can be selected freely as needed, a representative example may include providing a sealant layer for thermal adhesion or thermosealibility in the outermost layer and also combining with a polyolefin film, a polyester film, or the like according to the intended use.

Whey protein layer properties

The boundaries in oxygen barrier properties are difficult to define, especially because each market requires different levels of oxygen and humidity barriers. The film thickness can also be varied in order to compensate different intrinsic barrier efficiency.

For the evaluation of the barrier properties of the pilot scale products, the samples for the measurements of the Oxygen Transmission Rate (OTR) and the Water Vapour Transmission Rate (WVTR) are prepared in pieces of 20 x 20 cm and after inserted into the device for the analysis.

In the analysis for the OTR and the WVTR the sample works like a membrane between two different atmosphere: in both kinds of analysis the coated side is placed in the upper part of the chamber (pure oxygen for the OTR and high level humidity for the WVTR) to simulate the nearest condition to the reality.

In case of the OTR the coated side of the sheet is in the part where the pure oxygen passes and for the WVTR on the side of 85 % RH.

In every case the result of the analysis is recorded when the measured characteristic is fixed on a stable value (steady state).

Oxygen Transmission Rate

In this kind of trial as in the one for the WVTR the principle is to measure the amount of oxygen which is transferred trough the sample during a period in specific condition of temperature and relative humidity (23 °C and 50 % of relative humidity (RH)).

In the upper side of the chamber flows pure oxygen ($\geq$ 99,5%) and in the other side passes dry nitrogen (with 3% of hydrogen).

The oxygen is humidified to 50% before entering into the chamber and the carrier gas is purified with the use of a catalyst.

The reference measurement (zero) is made by fluxing pure nitrogen in the upper side of the chamber to have the same kind of flux and substance in the upper and in the lower half of the cell.

The film acts as a membrane and the transfer of the oxygen is due to the difference in partial pressure of it: the gas moves by diffusion into the film until the time that the driving force is equals constant and an equilibrium state is reached. The rate of oxygen is measured with a detector in the outgoing stream of the dry side and this is the value of the oxygen transmission rate to take in consideration. The final result is given in $cm^3/m^2$ d bar.

Oxygen and water vapour permeability values of whey-based coatings are converted to a thickness of 100 $\mu$m ($Q_{100}$) in order to allow direct comparison of different materials independently of the coating thickness. Film thicknesses were measured with the instrument Mahr Millimar C1216 of Mahr GmbH (Göttingen) after oxygen transmission tests. WPI coating thickness was calculated by subtracting the base for substrate film.

Water Vapour Transmission Rate

This trial permits to determine the WVTR: it measured the amount of water vapour passing trough the sample during a period of time in specific condition of temperature and gradient of relative humidity (23 °C and 85→0% of relative humidity).

The apparatus for the measurement of the WVTR is similar to the one for the OTR: here the dry nitrogen (carrier gas) sweeps the lower half of the chamber, in the upper side it is placed a porous frit soaked with a mixture of sulphuric acid and water.

In this half part of the chamber the RH (equals to 85%) is established on the ratio between the concentration of the acid and amount of water.

The nitrogen (carrier gas) is dried to 0% of RH before entering into the lower half part of the chamber by a desiccant. It takes the humidity permeated trough the sample and carries it to the sensor to trace the necessary current required for the electrolytic decomposition of the water. In this case the result is indicated as $g/m^2$d.

Mechanical properties

Additionally, the whey-protein coated substrate film obtainable by the process of the invention shows suitable thermo-mechanical properties, which are prerequisites for processability, and also shows suitable capability to withstand post operations and suitable use and durability.

The whey-protein coated substrate film obtainable by the process of the invention is capable of withstanding a substantial deformation (locally up to 400%) without crack initiation during the process at temperatures varying depending on the substrate. Additionally, thermal properties are also important because of filling conditions (e.g. hot filling possibility), post-packaging operations (possibly sterilisation, pasteurisation, microwaving of packed food etc.) and storage conditions (for freeze packed food). Adequate mechanical strength throughout the service life is necessary to ensure the integrity of the packaging.

The mechanical properties of the whey-protein coated film on a PET film obtainable by the process of the invention ranges as follows:

| Property | Range |
| --- | --- |
| Young modulus E (GPa) | 0.9-2.8 |
| Film Elongation at Break (%) | 80-420 |
| Stress yield (MPa) | 40-130 |
| Glass transition temperature (°C) | 122-139 |

The whey-protein coated film obtainable according to the process of the present invention shows suitable additional features such as those qualifying the aspect of the layer, scratch resistance, gloss, transparency, and surface finish after mechanical stress.

Adhesion

The bond strength measurement method measures the interlaminar strength which keeps together two different surfaces and was applied to the laminate samples (e.g. PET/Whey-protein layer/Adhesive/PE). The equipment is composed by the same machine and clamps used as for the commonly used tensile and the tear test (sample holder according to EN ISO 4624 and EN ISO 527-1). For each test, two samples with dimensions 100 mm per 15 mm are prepared and they are cut according to either the machine or the transverse direction. The two surfaces are then split up for a length of 40 mm and kept in constant conditions of 23°C and 50 % relative humidity. The ends of the samples are positioned into the clamps of the tensile machine and the bond strength is measured.

The adhesion between the whey layer and the substrate is measured according to the International Standard EN ISO 4624:2002 (pull-off test).

Coating systems and their viscosity requirements

According to an embodiment of the present invention, the coating process may be carried out by different coating technologies known in the prior art.

The coated film may go through the coating process various times for example to apply the whey-based surface layer after lamination of the whey-based barrier layer with the second structural layer, or by making successive coatings to increase the thickness of the barrier layer.

Different technologies, often classified as dry and wet processes could be envisaged for the formation of the whey-based films. In the case of the former, the proteins are heated above their glass transition temperature to form a film, whereas in the latter, the protein dispersion is applied to form a film (by spray, brush, coater, etc.).

According to an embodiment of the present invention, the coating process is carried out by a lacquering process.

Lacquering process

In this method the coating solution, comprising water, whey protein and plasticizers, is put inside a tub for its containment and with the help of a stainless steel roll (or of different suitable material) is deposited on the substrate. In this phase it is possible to adjust the wet thickness of the layer. After that the film is able to enter inside the drying tunnel.

The different techniques of coating depend on the viscosity of the solution that must be deposited. If the solution is not very viscous, like emulsions, it is better to use air knives, blades or bar coaters.

The roller coating process is preferable since it offers the most promising method for the industrialization purpose whereby the whey suspension is to be applied on the plastic substrates. Some variations of this process are described in the following paragraphs.

The reverse gravure coating system is based on an engraved roller immersed in a tank, where the coating material fills the roller engravings or slits. The coating is deposited on the substrate as it passes between the engraved roller and the pressure roller while excess material is removed by the doctor blade.

In the reverse roll coating technique, the coating material is measured onto the application roller thanks to precision setting of the gap between the metering roller lying above the application roller. The coating material is brushed off the application roller by the substrate as it passes around the bottom support roller.

Finally, in the Meyer bar coating process, an excess coating is deposited on the substrate by means of a roller immersed in a tank. A threaded steel bar (the Meyer bar) allows the required quantity of coating to remain on the substrate. The quantity is determined by the diameter of the threading on the bar. This coating system caters for wide tolerances in precision on the machines.

Table 2 summarizes the different coating systems and their rough viscosity ranges

Table 2.

| Application system | Description | Viscosity range Web speed |
|---|---|---|
| Roller application | Coating material is taken up from an application roller out of a bath and is either applied onto the substrate or onto a second roller (indirect process). | 1-10000 mPas |
| Gravure coating | Coating material is taken up from an engraved roller that runs in a bath and is applied onto the substrate that passes pressure roll and gravure roll. A doctor blade takes off the excess material, leaving only the required quantity of solution over the substrate. | 1 - 15 000 mPas |
| Comma rod | Coating material is applied to the substrate and the notch of the comma bar system acts as rod that removes the spare material | 100-50000 mPas |
| Air knife | Coating material is applied to the substrate and excess of coating on the substrate is removed by the use of a high velocity air jet. It's possible to obtain a uniform thickness of the coating layer. This technique is used with water base solution and it's not good with volatile solvent based solutions | 5 - 10000 mPas |
| Spray coating | Liquid is atomized into droplets which are sprayed onto the substrate. Viscosity controls size of the droplets. | 50 -150 mPas |
| Curtain coating | A continuous "curtain" of the coating material is established by a slot die. The substrate passes the curtain. | 10-5000 mPas 60 -1200 m/min |
| Slot coating | Coating material is squeezed through a slot onto the substrate. | 1-10000 mPas 1 - 600 m/min |

The native formulation can be applied at lower shear rate, preferably the method include via roller, airknife, curtain and slot coating.

Films coated according the process of the present invention with native whey proteins show the same good mechanical properties as those coated with denatured proteins. However, scratch resistance is rather low which allows reduction of the plasticizer to approximately half of the denatured reference formulation, and is not a problem when the coating is used as an interim layer. Additionally further crosslinking of the proteins takes place over time which strengthens the network.

Following the coating step, the whey-protein coated substrate film is dried and cured by heat treatment. Partial denaturation of the proteins occurs in this final step of the process.

The method of drying the coating liquid layer is not particularly limited, for example a hot roll contact technique, a heat medium (air, oil, and the like) contact technique, an infrared heating technique, a microwave heating technique, an ultraviolet heating technique, and the like. It is possible to use two or more than these drying methods simultaneously or at staggered times to improve drying efficiency but also protein denaturation and crosslinking.

Depending on the heating application mode it is possible to classify the dryers in two different classes:

- Direct dryers: here the hot gas comes in contact with the product. In this category they are included the drying tunnel and the spray drying. Into a drying tunnel a flow of hot air is used to move away the water from the product. If the material being dried is a film, unwinding zones can be used for its transport through the tunnel, or for example, if the material has the shape of slices a belt conveyor can be employed.

- Indirect dryers: in these the heat, from hot gas, steam or thermal fluids, doesn't enter in contact with the product that has to be dried. The heat from the hot fluid to the material is transferred by conduction through a surface. In this class are included rotary, cone, drum and tray dryer.

According to an embodiment of the present invention, the drying step is carried out in a drying tunnel. The drying tunnel may operate having an almost constant drying temperature or, alternatively, it is possible to design the drying tunnel in such a way that a variable cycle of temperature is applied along the tunnel. The drying tunnel may operate with different heating techniques simultaneously or sequentially as previously listed.

The drying temperature to be applied ranges from 60 °C to 160 °C, preferably from 100 °C to 140 °C Obviously, the denaturalization degree which can be achieved during the drying step, not only depends of the heating method and the temperature, but also on the drying time which is depending on the dryer length and the coating speed.

Lamination

The whey protein layer is able to serve as good oxygen barrier and can either serve as upper layer or as sandwich layer in a composite. After a lamination stage, it is possible to obtain proper composites with whey protein coated polymer films. Depending on the position the whey layer holds (upper layer or sandwich layer) it meets different demands. Besides this fact a suitable formulation can be chosen according to factors like packed good, product shelf life or consumer demands.

Post processes

Subsequently, the whey-protein coated film may undergoes post processes in order to be formed into packaging by e.g. thermosealing or thermoforming, and is then filled with the food before optionally going through post-packaging operations such as pasteurisation.

Therefore, the whey-protein coated substrate film obtainable by the process of the present invention is compatible with specific post process temperatures and parameters for thermo-sealing, thermoforming and post-packaging operations such as pasteurization, sterilization, vacuum packaging: no cracking, and no alteration of properties at long term.

Final product properties

According to an embodiment of the invention, the whey-protein based coated substrate improves barrier properties of the substrate it is applied on: lower than 20 $cm^3/m^2$ d bar for oxygen (23 °C, 50% rH) and lower than 50 $g/m^2$ d for water vapour permeation (23 °C, 85% to 0% rH) for protein on polymer film substrate like PET. Preferably, lower than 5 $cm^3/m^2$ d bar for oxygen (23 °C, 50% rH) and lower than 10 $g/m^2$ d for water vapour permeation (23 °C, 85% to 0% rH), more preferably lower than 1 $cm^3/m^2$ d bar for oxygen (23 °C, 50% rH) and lower than 2 $g/m^2$ d for water vapour permeation (23 °C, 85% to 0% rH). The above mentioned OTR and WVTR values are related to $Q_{100}$ values, i.e. normalized to a 100 $\mu$m thickness.

Although a thickness of the coating composition when laminated with the substrate is not particularly limited, it is preferably from 5 to 50 $\mu$m, more preferably between 7-20 $\mu$m. Greater thickness can be achieved by applying and drying several successive coating layers.

The definitions provided herein, within context, may be used exclusively, or may be used to supplement definitions which are generally known to those of ordinary skill in the art.

Throughout the description and claims the word "comprise" and variations of the word, such as "comprising", are not intended to exclude other technical features, additives, components, or steps.

Furthermore, the present invention covers all possible combinations of particular and preferred steps described hereinabove.

Additional objects, advantages and features of the invention will become apparent to those skilled in the art upon examination of the description or may be learned by practice of the invention. The following examples are provided by way of illustration, and are not intended to be limiting of the present invention.

DETAILED DESCRIPTION OF PARTICULAR EMBODIMENTS

Example 1. Preparation of the coating formulation.

Preparation of native coating formulations and pre-denatured coating formulation (comparative) was carried out as follows.

Whey protein isolate (WPI) BiPro of Davisco Foods International (Le Sueur) (dry protein pureness 97.4 %; N x 6.38) was used for formulating the whey-based coatings in the present study. Glycerol and sorbitol used as plasticizer were supplied by Merck Schuchard OHG (Hohenbrunn) and Merck KGkA (Darmstadt) respectively.

Preliminary trials showed that solutions with 12 wt. % whey protein isolate are gelatinizing during denaturation process (critical concentration at 12 wt. % was observed). For that reason further formulations were prepared using 10 wt. % WPI-solutions because they were easier to handle.

Pre-denatured protein formulations were prepared by heating aqueous WPI solutions (10 % w/w of the total mass of the solution) to 90°C for 30 min (above their temperature of denaturation of around 58-60°C as measured by DSC) using an electronic stirrer with heating, Thermomix 31-1, from Vorwerk Elektrowerk GmbH & CoKG (Wuppertal). After cooling the solutions to room temperature in a water bath, 10 wt. % of sorbitol (50 wt. % of the amount of dry matter based on the dry matter content of whey protein) was added and stirred for another 30 min (at 200 rpm). Degassing was performed via ultrasonication in each stage.

For native formulations heating is omitted, and a concentration of 20 wt. % of protein was used, and 10 wt. % of sorbitol based on the dry matter content of whey protein.

Since viscosity of the whey protein solution was a limiting factor of coating process, for comparison reasons 10 wt. % denatured whey protein and 20 wt. % native whey protein were used due to they show similar viscosity profile.

The amount of plasticizer is given as percentage and is always referred to the total amount of protein in the solution.

| Characteristics | Native coating formulation | Pre-denatured coating formulation |
|---|---|---|
| Viscosity [mPas] | 15 | 161 |
| Denaturation Enthalpy -dried film- (J/g) | 24.5 | 0 |
| Degree of denaturation (internal standard %) | 0 | 100 |

Commercial WPI BiPro was used as internal standard for maximum nativity, i.e. 100 % nativity was assumed.

Example 2. Pilot scale coating and drying

The machine used for the coating of the film and drying in a hot air continuous tunnel dryer was a customized Floatec / Rolltec-Highdry 250 model from Drytec, Hamburg-Nordstedt, Germany. The main parts of the machine are in order:

- Corona unit (surface pre-treatment of the substrate);
- Smooth roll application system (deposition of the coating solution on the substrate);
- Drying tunnel (where the solvent, in this case water, is removed);
- Controlled winding (in this zone is fundamental to have a perfect dry product to avoid the possibility that the 2 sides of the film will stick together).

After corona treatment (200 W) of the substrate surface, it was coated with the protein solution prepared according to process described in example 1.

The film after the lacquering step (and corona pre-treatment, 200 W) was entered into the machine and move progressively through the tunnel in contact with hot air. For the coating operation a gravure roll was used.

In the first section of the tunnel the hot air come only from the down side to heat the substrate and to remove the dirty particles and the air entrapped contained into the whey solution. In the second section the air flow comes from both sides of the machine and the drying of the whey layer starts from the upside too.

The hot air comes out from nozzles arranged all along the length of the machine to have a constant flow on the entire drying surface and to maintain constant the operation temperature too.

When the film got out from the machine all the water was evaporated and the solid layer deposited on the polymeric substrate was composed only by the proteins and the plasticizers.

The dryer provides 8000 m$^3$ / h of air and 1/4 of this volume was re-circulated.

The length of the drying tunnel was 4.2 m and the speed of the rolls which pull the substrate was 3 m/ min, this means that the drying time was 1.4 minutes in this example. Depending on the drying conditions (e.g. air velocity) the drying can be performed faster.

The resulting dry coating thickness ranges between 5 and 6 $\mu$m.

Example 3. Production of a laminate

The aim of this example was to obtain good barrier properties against oxygen and water vapour comparable to those obtained by using material such as Ethylene vinyl alcohol (EVOH) as barrier layer.

Two different laminated were produced: one using a coating solution of native whey proteins and the other using a coating solution of fully denatured whey proteins (solutions obtained as described in example 1).

For this sample a comma blade was used for the coating which was dried as described in example 2.

The first laminate was so formed using native whey protein solution:

- PET substrate (12 $\mu$m);
- Whey layer (5-6 $\mu$m);
- Adhesive (1-3 $\mu$m):
- PE (30 $\mu$m).

The PET was coated with native whey protein solution and dried at 140 °C, to obtain a significant denaturation of the proteins.

The adhesive solution was deposited on the PE always with the use of a gravure roll and then dried at a temperature of 60 °C.

This adhesive solution was composed of:

- Liofol UK 3640 / Härter UK 6800 in a relation of 50:1;
- Ethyl acetate as solvent.

The Liofol (300 g) was initially mixed with Ethyl acetate (434 g) and later the Härter (6 g) was added at the solution. It must be considered that 1 kg of solution Liofol / Härter and 1.42 kg of solvent give a final solution with 30 wt. % of dry matter content.

The coated PET and the coated PE were put inside the lacquering machine and laminated with use of two cylinders which turn in opposite direction (the lamination zone is arranged in the last part of the machine, near the unwinding unit).

For the production of a laminated product using denatured whey protein solution, the same procedure was used. The substrate was coated with the solution of fully denatured proteins and dried at a temperature of 105 °C (temperature considered sufficient for have a perfect drying process). The residual moisture is the same range in the 2 processes (3.3% for native and 3.6% for predenatured formulation) showing that the slight difference in temperature did not affect the drying efficiency.

Example 4. Evaluation of the barrier properties.

The samples for the measurements of the OTR and the WVTR were prepared in pieces of 20 x 20 cm and after inserted into the device for the analysis as described in the barrier properties measurement description.

Values of denaturation enthalpy of the coated sheets prepared according to example 2:

| Temperature of coating (°C) | DenaturationEnthalpy (J/g) |
| --- | --- |
| 23 | 24,493 |
| 60 | 19,310 |
| 80 | 18,435 |
| 100 | 17,805 |
| 120 | 17,354 |
| 140 | 16,945 |

The degree of denaturation is calculated taking as zero denaturation the value of denaturation enthalpy of the sheet dried at 23 °C:

$$Den\% = 100 - \left( \frac{Den_i}{Den_{23}} * 100 \right)$$

where $Den_x$ and $Den_{23}$ are respectively the value of the denaturation enthalpy at the general x drying temperature and 23 °C.

| Temperature | Amount of denaturation(%) |
|---|---|
| 23 | 0 |
| 60 | 21,16 |
| 80 | 24,74 |
| 100 | 27,31 |
| 120 | 29,15 |
| 140 | 30,82 |

OTR was measured according to DIN 53380-3 (DIN, 1998) with an instrument of Brugger Feinmechanik GmbH which was located in an air-conditioned laboratory at 23 °C. The coated film was placed between two measuring cells. After rinsing the two chambers with nitrogen to determine the reference value (in case of any leakage), a stream of oxygen was passed through the first chamber whereas the other chamber was purged with nitrogen as carrier gas. To guarantee that only pure nitrogen passes through the second chamber nitrogen and 2 % of hydrogen was induced. At the catalyst hydrogen reacts in case of presence of oxygen and forms water. For creating RH of 50 % carrier gas and oxygen was passed through humidifiers. Over time oxygen passes the film and is dissolved in the carrier gas which passes a detector. The electrochemical detector enables oxygen molecules to react at the graphite-cathode and the cadmium-anode (saturated in caustic potash) and to generate electrical current that is proportional to the amount of oxygen.

The measurement was finished when a steady state (for at least 10 hours) was obtained. As OTR value the difference between measured value and reference value is stated in terms of $cm^3/m^2 \cdot d \cdot bar$.

Two samples were determined and the average value was used for further calculations like $Q_{100}$. In case of deviation > 10 % a third determination was done. RH on both sides of the film was 50 %.

The following table contains the denaturation enthalpy, degree of denaturation after drying, WVTR and OTR of the products obtained as described in example 2:

| Drying $T^a$ | Denaturation Enthalpy [J/g] | Degree of denaturation (internal standard) [%] | WVTR (100$\mu$m) [g/(m²d)] at 23°C; 85 → 0% RH | OTR (100$\mu$m) [cm³/(m²d bar)] at 23°C; 50% RH |
|---|---|---|---|---|
| 23°C (native) | 24,5 | 0 | 303,0 | 27,9 |
| 100°C (native) | 17,4 | 27 | 3,2 | 4,6 |
| 140°C (native) | 16,9 | 31 | 2,5 | 1,3 |
| Pre-denatured formulation (95°C - 30min) | 0 | 100 | 2,3 | 1,3 |

The degree of denaturation is calculated taking as zero denaturation the value of denaturation enthalpy of the dried sheet at 23°C (internal standard).

The higher nativity of the proteins used can be confirmed considering the respective denaturation enthalpy which decreases by applying higher drying temperatures. It is possible to achieve similar OTR and WVTR values compared with pre-denatured protein formulations.

Using the laminate bond strength method, it was not possible to separate the layers since the substrate (PET) broke at 5.5 - 6 N / 15 mm earlier. Therefore the adhesion measurement method according to the International Standard EN ISO 4624:2002 (pull-off test) was performed.

Results of the Pull-off test showed that whey-based layers display excellent adhesion due to the corona pre-treated substrates where it was applied with peeling forces over the standard as only cohesive failures in the substrates were observed as opposite to adhesive fractures at the whey-based layer / substrate interface. It can be concluded that the average cracking load was 2 N.

**Claims**

1. A process for preparing a whey-protein coated substrate film for packaging, comprising the following steps:

   a) providing a coating composition comprising a water solution of whey protein, which has at least 40% in its native state, the whey protein being selected from the group consisting of a whey protein isolate, a whey protein concentrate, and a mixture thereof; and
   b) applying directly the coating composition of step a) onto a substrate film in order to obtain a coated substrate film wherein the coating has at least 40% of the whey protein in its native state; and
   c) drying it at a temperature between 60-160 °C.

2. The process according to claim 1, wherein the coating composition has a dry matter content between 5-75 wt. %.

3. The process according to any of the claims 1-2, further comprising the addition of a plasticizer selected from the group consisting of polyethylene glycol, propyleneglycol, glycerol and sorbitol to the coating composition of step a), wherein the plasticizer is present in the coating composition in an amount between 20 - 200 wt. % based on the dry matter content of whey protein.

4. The process according to any of the claims 1-3, further comprising a surface pretreatment of the substrate, selected from the group consisting of corona discharge and plasma treatment before applying the coating composition of step a) onto the substrate film.

5. The process according to any of the claims 1-4, wherein the coating composition of step a) further comprises other additives selected from anti-oxidants, antimicrobials, colorants, pigments, ultraviolet absorbers, antistatic agents, crosslinkers, fillers, oxygens scavengers, humidity absorbers, biocides, and mixtures thereof.

6. The process according to any of the claims 1-5, further comprising a previous step wherein the whey protein is submitted to an acetylation modification, a succinylation modification, an enzymatic modification or to a high pressure homogenization.

7. The process according to claim 6, wherein the modification is carried out by treatment with acetic anhydre, succinic anhydre or a protease enzyme.

8. A whey-protein coated substrate obtainable by the process according to any of the claims 1-7.

9. The whey-protein coated substrate according to claim 8, having lower than 20 $cm^3/m^2$ d bar of oxygen transmission rate measured at 23 °C and 50% relative humidity; lower than 50 $g/m^2$ d for water vapour permeation rate measured at 23 °C and 85% to 0% relative humidity; oxygen permeation values are normalized to a 100 $\mu$m thickness; and wherein the thickness of the coating composition when laminated with the substrate is comprised from 5 to 50 $\mu$m; and the substrate is PET.

10. The whey-protein coated substrate according to any of the claims 8-9, wherein the coating composition comprises sorbitol as a plasticiser and the whey protein content is between 15-25 % w/w.

11. The whey-protein coated substrate according to any of the claims 8-9, wherein the coating composition comprises glycerol as a plasticiser and the whey protein content is between 20-35 % w/w.

12. Packaging film comprising at least one or more of the whey protein coated substrate as defined in any of the claims 8-11.

13. Use of whey protein coated substrate as defined in any of the claims 8-11 as packaging film.

**14.** A food, pharmaceutical or cosmetic product packaged in a packaging film as defined in claim 12.


**Patentansprüche**

**1.** Ein Verfahren zur Herstellung eines mit Molkenprotein beschichteten Substratfilms für die Verpackung, die folgenden Schritte umfassend:

a) Bereitstellen einer Beschichtungszusammensetzung, eine wässrige Lösung von Molkenprotein umfassend, die mindestens 40 % in ihrem nativen Zustand aufweist, wobei das Molkenprotein aus der Gruppe bestehend aus einem Molkenproteinisolat, einem Molkenproteinkonzentrat und einer Mischung davon ausgewählt ist; und
b) direktes Auftragen der Beschichtungszusammensetzung aus Schritt a) auf einen Substratfilm, um einen beschichteten Substratfilm zu erhalten, worin die Beschichtung mindestens 40 % des Molkenproteins in seinem nativen Zustand aufweist; und
c) Trocknen bei einer Temperatur zwischen 60-160 °C.

**2.** Das Verfahren nach Anspruch 1, worin die Beschichtungszusammensetzung einen Trockenmassegehalt zwischen 5-75 % aufweist.

**3.** Das Verfahren nach einem der Ansprüche 1-2, ferner die Zugabe eines Weichmachers umfassend, ausgewählt aus der Gruppe bestehend aus Polyethylenglykol, Propylenglykol, Glycerin und Sorbitol zu der Beschichtungszusammensetzung von Schritt a), worin der Weichmacher in der Beschichtungszusammensetzung in einer Menge zwischen 20 und 200 % bezogen auf den Trockenmassegehalt von Molkenprotein vorhanden ist.

**4.** Das Verfahren nach einem der Ansprüche 1-3, ferner eine Oberflächenvorbehandlung des Substrats umfassend, ausgewählt aus der Gruppe bestehend aus Koronaentladung und Plasmabehandlung, bevor die Beschichtungszusammensetzung aus Schritt a) auf den Substratfilm aufgebracht wird.

**5.** Das Verfahren nach einem der Ansprüche 1-4, worin die Beschichtungszusammensetzung von Schritt a) ferner andere Additive umfasst, die ausgewählt sind aus Antioxidantien, antimikrobiellen Mitteln, Farbmitteln, Pigmenten, Ultraviolettabsorbern, Antistatika, Vernetzern, Füllstoffen, Sauerstofffängern, Feuchtigkeitsabsorbern, Bioziden und Mischungen derselben.

**6.** Das Verfahren nach einem der Ansprüche 1-5, ferner umfassend einen vorherigen Schritt, worin das Molkenprotein einer Acetylierungsmodifikation, einer Succinylierungsmodifikation, einer enzymatischen Modifikation oder einer Hochdruckhomogenisierung unterzogen wird.

**7.** Verfahren nach Anspruch 6, worin die Modifikation durch Behandlung mit Essigsäureanhydrid, Bernsteinsäureanhydrid oder einem Protease-Enzym durchgeführt wird.

**8.** Ein mit Molkenprotein beschichtetes Substrat, erhältlich durch das Verfahren nach einem jeden der Ansprüche 1-7.

**9.** Ein mit Molkenprotein beschichtetes Substrat nach Anspruch 8 mit weniger als 20 cm°/m" d bar Sauerstoffdurchlässigkeitsrate, gemessen bei 23 °C und 50 %, relative Luftfeuchtigkeit; weniger als 50 g/m d für die Wasserdampfpermeationsrate gemessen bei 23 °C und 85 % bis 0 % relativer Luftfeuchtigkeit; Sauerstoffpermeationswerte sind auf eine Dicke von 100 p m normiert; und wobei die Dicke der Beschichtungszusammensetzung, wenn sie mit dem Substrat laminiert ist, von 17 bis 50 pm umfasst; und das Substrat PET ist.

**10.** Das mit Molkenprotein beschichtete Substrat nach einem der Ansprüche 8-9, worin die Beschichtungszusammensetzung Sorbitol als ein Weichmacher umfasst und der Molkenproteingehalt zwischen 15-25 % liegt.

**11.** Das mit Molkenprotein beschichtete Substrat nach einem der Ansprüche 8-9, worin die Beschichtungszusammensetzung Glyzerin als ein Weichmacher umfasst und der Molkenproteingehalt zwischen 20-35 % liegt.

**12.** Verpackungsfolie, mindestens ein oder mehrere des mit Molkenprotein beschichteten Substrats umfassend, wie in einem der Ansprüche 8-11 definiert.

**13.** Verwendung eines mit Molkenprotein beschichteten Substrats, wie in einem der Ansprüche 8-11 als Verpackungsfolie definiert.

14. Ein Lebensmittel, pharmazeutisches oder kosmetisches Produkt, verpackt in einer Verpackungsfolie, wie in Anspruch 12 definiert.

**Revendications**

1. Procédé de préparation d'un film de substrat revêtu de protéine de lactosérum pour le conditionnement, comprenant les étapes suivantes :

   a) fournir une composition de revêtement comprenant une solution aqueuse de protéine de lactosérum, qui a au moins 40 % dans son état d'origine, la protéine de lactosérum étant choisie dans le groupe consistant en un isolat de protéine de lactosérum, un concentré de protéine de lactosérum et un mélange de ceux-ci ; et
   b) appliquer directement la composition de revêtement de l'étape a) sur un film de substrat afin d'obtenir un film de substrat revêtu dans lequel le revêtement a au moins 40 % de la protéine de lactosérum à l'état d'origine ; et
   c) le faire sécher à une température comprise entre 60-160 °C.

2. Procédé selon la revendication 1, dans lequel la composition de revêtement a une teneur en matière sèche comprise entre 5-75 % en poids.

3. Procédé selon l'une quelconque des revendications 1-2, comprenant en outre l'ajout d'un plastifiant choisi dans le groupe consistant en polyéthylène glycol, propylène glycol, glycérol et sorbitol à la composition de revêtement de l'étape a), dans lequel le plastifiant est présent dans la composition de revêtement en une quantité comprise entre 20 - 200 % en poids sur la teneur en matière sèche de la protéine de lactosérum.

4. Procédé selon l'une quelconque des revendications 1-3, comprenant en outre un prétraitement de surface du substrat, choisi dans le groupe consistant en la décharge de corona et le traitement au plasma avant d'appliquer la composition de revêtement de l'étape a) sur le film de substrat.

5. Procédé selon l'une quelconque des revendications 1-4, dans lequel la composition de revêtement de l'étape a) comprend en outre d'autres additifs choisis parmi les antioxydants, les antimicrobiens, les colorants, les pigments, les absorbeurs d'ultraviolets, les agents antistatiques, les agents de réticulation, les charges, les récupérateurs d'oxygène, les absorbeurs d'humidité, les biocides, et des mélanges de ceux-ci.

6. Procédé selon l'une quelconque des revendications 1-5, comprenant en outre une étape précédente dans laquelle la protéine de lactosérum est soumise à une modification d'acétylation, une modification de la succinylation, une modification enzymatique ou à une homogénéisation à haute pression.

7. Procédé selon la revendication 6, dans lequel la modification est effectuée par traitement avec de l'anhydride acétique, de l'anhydre succinique ou une enzyme protéase.

8. Substrat revêtu de protéine de lactosérum pouvant être obtenu par le procédé selon l'une quelconque des revendications 1-7.

9. Substrat revêtu de protéine de lactosérum selon la revendication 8, ayant un taux de transmission d'oxygène inférieur à 20 cm3/m2 dbar mesuré à 23 °C et 50 % d'humidité relative ; inférieur à 50 g/m2 d pour le taux de perméation de vapeur d'eau mesuré à 23 °C et une humidité relative comprise entre 85 % et 0 % ; les valeurs de perméation d'oxygène sont normalisées à une épaisseur de 100 $\mu$m ; et dans lequel l'épaisseur de la composition de revêtement lorsqu'elle est laminée avec le substrat est comprise entre 5 et 50 $\mu$m ; et le substrat est PET.

10. Substrat revêtu de protéine de lactosérum selon l'une quelconque des revendications 8-9, dans lequel la composition de revêtement comprend du sorbitol en tant que plastifiant et la teneur en protéine de lactosérum est comprise entre 15-25 % p/p.

11. Substrat revêtu de protéine de lactosérum selon l'une quelconque des revendications 8-9, dans lequel la composition de revêtement comprend du glycérol en tant que plastifiant et la teneur en protéine de lactosérum est comprise entre 20-35 % p/p.

**12.** Film de conditionnement comprenant au moins un ou plusieurs substrats revêtus de protéine de lactosérum tels que définis dans l'une quelconque des revendications 8-11.

**13.** Utilisation de substrat revêtu de protéine de lactosérum tel que défini dans l'une quelconque des revendications 8-11, en tant que film de conditionnement.

**14.** Produit alimentaire, pharmaceutique ou cosmétique conditionné dans un film de conditionnement tel que défini dans la revendication 12.

## REFERENCES CITED IN THE DESCRIPTION

**Patent documents cited in the description**

- US 5543184 A

**Non-patent literature cited in the description**

- *J Agricultural and Food Chem,* 2006, vol. 54/19, 7152-7158
- *J Food Sci,* 1999, vol. 64/6, 1034-1037
- *Pakistan J of Nutr,* 2011, vol. 10/3, 296-301
- *J Dairy Sci,* 2014, vol. 97, 5315-5327
- *Critical Rev in Food Sci and Nutrition,* 1993, vol. 33 (6), 431-476